# EUROPÄISCHE PATENTSCHRIFT

(11) **EP 1 302 456 B1**
(45) Veröffentlichungstag und Bekanntmachung des Hinweises auf die Patenterteilung: **02.01.2004**
(21) Anmeldenummer: 02022244.4
(22) Anmeldetag: 02.10.2002
(51) Int. Cl.: C07B 41/06, C07C 45/30, C07C 47/198, C07D 209/48

(54) **Verfahren zur Oxidation von Alkoholen unter Katalyse von Nitroxylverbindungen**
Process for oxidation of alcohols catalysed by nitroxyl compounds
Procédé d'oxidation des alcohols catalysés par des composés nitroxyls

(30) Priorität: 11.10.2001 DE 10150164; 15.11.2001 DE 10156138
(43) Veröffentlichungstag der Anmeldung: 16.04.2003
(73) Patentinhaber: Consortium für elektrochemische Industrie GmbH, 81379 München (DE)
(72) Erfinder: Fritz-Langhals, Elke, Dr., 85521 Ottobrunn (DE); Petersen, Hermann, Dr., 84489 Burghausen (DE); Stohrer, Jürgen, Dr., 82049 Pullach (DE)
(74) Vertreter: Potten, Holger

(56) Entgegenhaltungen:
- ANELLI P L ET AL: "FAST AND SELECTIVE OXIDATION OF PRIMARY ALCOHOLS TO ALDEHYDES OR TOCARBOXYLIC ACIDS AND OF SECONDARY ALCOHOLS TO KETONES MEDIATED BY OXOAMMONIUM SALTS UNDER TWO-PHASE CONDITIONS" JOURNAL OF ORGANIC CHEMISTRY, AMERICAN CHEMICAL SOCIETY. EASTON, US, Bd. 52, Nr. 12, 12. Juni 1987 (1987-06-12), Seiten 2559-2562, XP000567759 ISSN: 0022-3263
- INOKUCHI T ET AL: "A SELECTIVE AND EFFICIENT METHOD FOR ALCOHOL OXIDATIONS MEDIATED BY N-OXOAMMONIUM SALTS IN COMBINATION WITH SODIUM BROMITE" JOURNAL OF ORGANIC CHEMISTRY, AMERICAN CHEMICAL SOCIETY. EASTON, US, Bd. 55, Nr. 2, 19. Januar 1990 (1990-01-19), Seiten 462-466, XP000567758 ISSN: 0022-3263

## Beschreibung

Die vorliegende Erfindung beinhaltet ein kontinuierliches Verfahren zur Oxidation von Alkoholen mit Hilfe von Nitroxylverbindungen als Katalysatoren in mehrphasigen Systemen.

Die Oxidation von Alkoholen zu Aldehyden bzw. Ketonen ist eine wichtige Transformation in der organischen Chemie, da aus den gut zugänglichen Alkoholen Verbindungen mit einem hohen Reaktionspotential gebildet werden. Solche Transformationen sind daher auch in technischen Prozessen von großer Bedeutung. Von Vorteil sind insbesondere katalytische Verfahren. Ein häufig technisch angewendetes Verfahren ist die Gasphasendehydrierung von Alkoholen, wobei allerdings nur flüchtige Verbindungen eingesetzt werden können. Die verwendeten Katalysatorsysteme sind jeweils auf wenige Substrate beschränkt, die Reaktionsbedingungen müssen speziell angepasst werden. Allgemeiner anwendbar sind Oxidationen unter Verwendung von Nitroxylverbindungen als Oxidationskatalysatoren, insbesondere von TEMPO (2,2,6,6,-Tetramethylpiperidin-1-oxyl) und seinen Derivaten (Übersicht: A.E.J. de Nooy, A.C. Besemer und H.V. Bekkum, Synthesis 1996, 1153). Zahlreiche TEMPO-Derivate sind als Oxidationskatalysatoren beschrieben, darunter auch TEMPO-Derivate auf polymeren Trägern, z. B. PIPO (polyamine immobilised piperidinyl oxyl, Dijksman et al., Synlett 2001, 102, EP 1103537). Häufig werden diese TEMPO-katalysierten Oxidationen in Zweiphasensystemen, z. B. Methylenchlorid / Wasser, durchgeführt (P.L. Anelli, C. Biffi, F. Montanari und S. Quici, J. Org. Chem. 1987, 52, 2559). Gut untersucht ist die Oxidation von Alkoholen mit Natriumhypochlorit oder Natriumhypobromit als Oxidationsmittel. Nach diesem Verfahren können aus Alkoholen Aldehyde und aus sekundären Alkoholen Ketone gewonnen werden.

Bei der üblichen Durchführung der Synthesen im Batch-Verfahren wird das in der Wasserphase gelöste Oxidationsmittel der organischen Phase, die den zu oxidierenden Alkohol und die Nitroxylverbindung enthält, zugesetzt. Ein Nachteil dieser bestehenden Verfahrensweise ist, dass die Reaktionswärme des Nitroxyl-katalysierten stark exothermen Oxidationsprozesses insbesondere bei großen Ansätzen nur sehr schwer abgeführt werden kann. Dies erhöht die Kontaktzeit der beiden Phasen und damit die Prozessdauer, wodurch Nebenreaktionen, z.B. die Reaktion Alkali-labiler Gruppen, etwa die Verseifung von Estergruppen, wesentlich stärker in den Vordergrund treten.
Als Kontaktzeit wird hier die Zeit verstanden, in der die an der Reaktion beteiligten Phasen durchmischt werden.
Eine weitere Nebenreaktion bei Batch-Versuchen ist die Bildung von Halbacetalen mit dem als Edukt verwendeten Alkohol und dessen Weiteroxidation zum Ester nach Gl. 1.

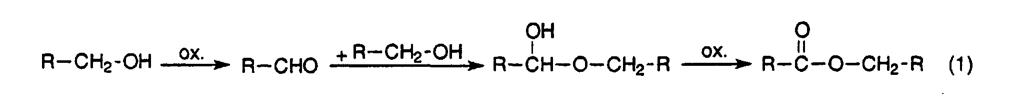

Mit zunehmender Reaktionsdauer und Phasenkontaktzeit tritt diese Reaktion immer weiter in den Vordergrund, so dass die erzielbaren Ausbeuten mit zunehmender Ansatzgröße sinken und eine technische Produktion unmöglich machen. Eine weitere Nebenreaktion, die die Ausbeute des gebildeten Aldehyds mindert, ist die Weiteroxidation des gebildeten Aldehyds zur Carbonsäure.
Bei der Umsetzung von 20 g 2-n-Butyryloxyethanol enstanden nur 37 % des gewünschten Aldehyds neben 12 % 2-n-Butyryloxyessigsäure-2-n-butyryloxyethylester, 4 % Buttersäure und 41 % 2-n-Butyryloxyessigsäure (Vergleichsbeispiel 9). Durch Verdopplung der Ansatzgröße verringerte sich die Ausbeute an Aldehyd auf 12 %; die Menge an 2-n-Butyryloxyessigsäure-2-n-butyryloxyethylester betrug bereits 45 % (Vergleichsbeispiel 10).

In EP0340703 und in DE 4007923 werden TEMPO-katalysierte Oxidationsreaktionen mit Bleichlauge im Batch-Betrieb beschrieben. Die Kontaktzeiten liegen bei mehr als 15 min. bzw. mehr als 30 min, denn allein für die Nachreaktion wurden diese Zeiten eingehalten. Die Möglichkeit der Ausführung dieses Prozesses als kontinuierlicher Prozess wurde zwar in Betracht gezogen, aber nicht ausgeführt.

In DE 10029597 wird ein Oxidationsverfahren unter Verwendung eines polymervergrößerten TEMPO-Derivats ebenfalls als Batch-Prozess beschrieben. Die Möglichkeit einer kontinuierlichen Prozessführung wird nur in Verbindung mit der Verwendung eines Membranreaktors gesehen, aber nicht ausgeführt. Auch hier wird stets von Kontaktzeiten von mindestens 30 min. ausgegangen. DE 69415345 (EP 0734392) geht von Kontaktzeiten von 5 Std. aus. In der DE 19605039 (EP 0801073) wird von einer Kontaktzeit von mindestens 45 min ausgegangen. In der EP 1103537 beträgt allein die Nachreaktionszeit 20 min. In der WO 01/90111 sind Kontaktzeiten-von sogar 4 Std. eingehalten worden, allein für die Nachreaktion wurden 2 Std. aufgewendet.

Es zeigte sich, dass die bloße Übertragung des Batch-Prozesses auf ein kontinuierliches Verfahren die Ausbeuten nicht verbessert. Zwar wird dann keine Esterbildung beobachtet, jedoch tritt die Weiteroxidation des Aldehyds zur Carbonsäure verstärkt auf. Die bloße Übertragung des Batch-Verfahrens auf ein kontinuierliches Verfahren stellt daher keine Lösung der Ausbeuteproblematik dar. Dies ist auch aus den Vergleichsbeispielen 11 und 12 der vorliegenden Anmeldung ersichtlich.

Aufgabe der Erfindung ist es daher, ein Verfahren zur Oxidation eines Alkohols zu einem Aldehyd oder einem Keton unter Katalyse einer Nitroxylverbindung zur Verfügung zu stellen, das höhere Ausbeuten an Aldehyden oder Ketonen liefert.

Die Aufgabe wird gelöst durch ein Verfahren, bei welchem der zu oxidierende Alkohol in einer organischen flüssigen Phase in Gegenwart einer Nitroxylverbindung mit einer das Oxidationsmittel enthaltenden wässrigen Phase umgesetzt wird, dadurch gekennzeichnet, dass die Umsetzung mit einer Kontaktzeit der Phasen zwischen 0,1 s und maximal 15 min. und bei einer intensiven Durchmischung der Phasen kontinuierlich erfolgt.

Für das erfindungsgemäße Verfahren ist die Kombination der kontinuierlichen Prozessführung mit Kontaktzeiten unter 15 min wesentlich. Nur durch die Einhaltung beider Verfahrensmerkmale werden die oben beschriebenen Nebenreaktionen effizient unterdrückt.

Die für das Verfahren benötigten kontinuierlichen Reaktoren sind dem Fachmann bekannt. Eine Übersicht über die wichtigsten Ausführungsformen gibt z.B. "Ullmann's Encyclopedia of Industrial Chemistry", Vol. B4.

Das beanspruchte Verfahren kann beispielsweise in kontinuierlich betriebenen Rohrreaktoren, in kontinuierlich betriebenen Loop-Reaktoren, in kontinuierlich betriebenen Rührkesseln oder Rührkesselkaskaden oder mit Hilfe von Kreiselpumpen durchgeführt werden.

In einer besonders bevorzugten Ausführungsform können die beiden Phasen in einem statischen Mischelement zusammengeführt und durch einen Rohrreaktor weitergeleitet werden.

Die Kontaktzeit der Phasen beträgt bevorzugt 1 s bis 5 min, besonders bevorzugt 1s bis 2 min, ganz besonders bevorzugt 1s bis 30s.

Die intensive Durchmischung der Phasen wird bevorzugt erreicht durch die Ausbildung einer turbulenten Strömung in der Reaktionsmischung.

Besonders bevorzugt ist eine turbulente Strömung mit Werten für die Reynolds'sche Zahl Re zwischen 800 und 20000.

Eine intensive Durchmischung bzw. eine turbulente Strömung kann prinzipiell mit allen bekannten Mischsystemen, z.B. statischen Mischelementen oder Rührern, erzeugt werden. Zur Erreichung bzw. Überschreitung der Reynolds'schen Zahl können auch verschiedene Mischsysteme kombiniert werden.

Die organische flüssige Phase besteht aus dem Alkohol und gegebenenfalls einem oder mehreren organischen Lösungsmitteln. Als organische Lösungsmittel seien beispielhaft genannt lineare oder verzweigte gesättigte oder ungesättigte aliphatische Kohlenwasserstoffe mit 1-20 C-Atomen, zyklische aliphatische gesättigte oder ungesättigte Kohlenwasserstoffe mit 5-20 C-Atomen oder aromatische Kohlenwasserstoffe mit 5-20 C-Atomen, wobei ein Wasserstoff oder mehrere Wasserstoffe oder ein Kohlenstoff oder mehrere Kohlenstoffe durch Heteroatome ersetzt sein können.

Bevorzugt handelt es sich um lineare oder verzweigte gesättigte oder ungesättigte aliphatische Kohlenwasserstoffe mit 1-16 C-Atomen, zyklische aliphatische gesättigte oder ungesättigte Kohlenwasserstoffe mit 5-16 C-Atomen oder aromatische Kohlenwasserstoffe mit 6-16 C-Atomen,
wobei ein Wasserstoff oder mehrere Wasserstoffe unabhängig voneinander durch F, Cl, Br, NO₂ oder CN ersetzt sein können, oder wobei eine CH₂-Gruppe oder mehrere CH₂-Gruppen unabhängig voneinander durch O, NH, C=O, S, S=O, SO₂, P=O ersetzt sein können,
oder eine CH-Gruppe oder mehrere CH-Gruppen unabhängig voneinander durch N oder P ersetzt sein können,
oder quartäre Kohlenstoffe durch Si ersetzt sein können.

Beispiele für organische Lösungsmittel sind Hexan, Petrolether, Cyclohexan, Dekalin, Methylenchlorid, Chloroform, Tetrachlorkohlenstoff, 1,2-Dichlorethan, Chlorbenzol, Benzol, Toluol, 1-Chlornaphthalin, Essigsäureethylester, Essigsäurebutylester, Ethylencarbonat, Tetrahydro-1,3-dimethyl-2(1H)-pyrimidinon (DMPU), Hexamethylphosphorsäuretriamid (HMPT), Dimethylsulfoxid (DMSO), Diethylether, Methyl-tert-butylether, Ethylenglykoldiethylether, Diethylenglykoldiethylether, Dioxan, Diisopropylketon und Polydimethylsiloxane.

Der zu oxidierende Alkohol kann in Konzentrationen zwischen 0,1 und 100 Gew. % bezogen auf die organische Lösung, bevorzugt zwischen 1 und 50 Gew. % eingesetzt werden.

Unter dem Begriff Nitroxylverbindung sind ebenfalls die oxidierte Form des Oxoammoniumions und die reduzierte Form des Hydroxylamins zu verstehen, da diese Formen bei der Oxidation ebenfalls auftreten (A.E.J. de Nooy, A.C. Besemer und H.V. Bekkum, Synthesis 1996, 1155).

Bei der als Oxidationskatalysator eingesetzten Nitroxylverbindung handelt es sich vorzugsweise um eine Di-tertalkylnitroxylverbindung.

Bevorzugt handelt es sich um eine Nitroxylverbindung der allgemeinen Formel I 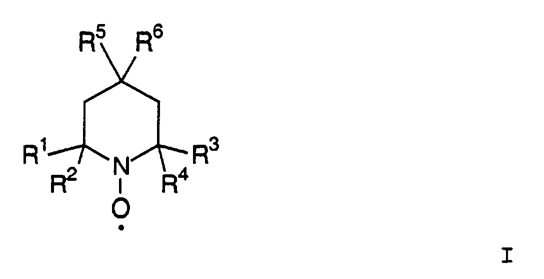 worin die Reste R¹, R², R³ und R⁴ unabhängig voneinander C₁-C₁₂-Alkyl oder C₂-C₁₂-Alkenyl oder C₆-C₁₂-Aryl oder Aralkyl bedeuten, und die Reste R⁵ und R⁶ unabhängig voneinander folgende Bedeutung haben: Wasserstoff, OH, CN, Halogen,
linear oder verzweigt, gesättigt oder ungesättigt C₁-C₂₀-Alkyl-, C₆-C₂₀-Aryl, C₆-C₂₀-Hetaryl oder C₆-C₂₀-Aralkyl, OR⁷, O-COR⁷, O-COOR⁷, OCONHR⁷ ,
COOH, COR⁷, COOR⁷, CONHR⁷ wobei R⁷ die Bedeutung eines linearen oder verzweigten, gesättigten oder ungesättigten C₁-C₂₀-Alkylrestes, oder die Bedeutung eines C₆-C₂₀-Aryl, C₃-C₂₀-Hetaryl oder C₆-C₂₀-Aralkylrests hat,
-(O-CH₂-CH₃)ₙ-OR⁸, -(O-C₃H₆)ₙ-OR⁸, -(O-(CH₂)₄)ₙ-OR⁸, -O-CH₂-CHOH-CH₂-(O-CH₂-CH₂-)ₙ-OR⁸ mit R⁸ in der Bedeutung Wasserstoff, C₁-C₂₀-Alkyl, C₆-C₂₀-Aralkyl, mit n = 1 bis 100 , oder CH₂-CHOH-CH₃ oder CH₂-CHOH-CH₂-CH₃ ,
NR⁹R¹⁰, NHCOR⁹, NHCOOR⁹, NHCONHR⁹, mit R⁹ und R¹⁰ unabhängig voneinander in der Bedeutung eines linearen oder verzweigten, gesättigten oder ungesättigten C₁-C₂₀-Alkylrestes, eines C₆-C₁₂-Cycloalkylrests, oder eines C₆-C₂₀-Aryl , C₃-C₂₀ Hetaryl oder C₆-C₂₀-Aralkylrests,
wobei die Reste R⁵ und R⁶ auch zu einem Ring verknüpft sein können,
und wobei die Reste R⁵ und R⁶ ihrerseits auch mit COOH, OH, SO₃H, CN, Halogen, prim., sek., tert. Amino oder quart. Ammonium substituiert sein können, oder
die Reste R⁵ und R⁶ zusammen auch die Bedeutung =O, =NR¹¹, =N-OR¹¹, =N-N=CR¹¹R¹² mit R¹¹ und R¹² unabhängig in der Bedeutung Wasserstoff, C₁-C₂₀-Alkyl oder C₆-C₂₀-Aralkyl haben können. Weiterhin bevorzugt handelt es sich bei der Nitroxylverbindung um zwei Moleküle der Formel I, die über eine Brücke =N-N= in 4-Position verknüpft sind.

Weiterhin bevorzugt handelt es sich bei der Nitroxylverbindung um zwei oder mehrere Moleküle der allgemeinen Formel I, die über einen der beiden Reste R⁵ oder R⁶ miteinander verbunden sind.
Dabei eignet sich als verknüpfender Rest beispielsweise O-Alkyl-O, O-CH₂-Aryl-CH₂-O, O-CO-NH-Aryl-NH-CO-O, O-CO-NH-Alkyl-NH-CO-O oder eine Brücke der allgemeinen Formel (O-(CH₂)ₙ-O)ₘ mit n = 2 bis 4 und m = 2 bis 50.
In einer weiteren Ausführungsform handelt es sich bei der Nitroxylverbindung um eine polymere Struktur enthaltend Verbindungen der Formel I, die über die Reste R⁵ oder R⁶ oder R⁵ und R⁶ verknüpft ist. In der Lit. ist eine Vielzahl solcher Strukturen beschrieben (EP 1103537, Cirriminna et al., Chem. Commun. 2000, 1441; Bolm et al., Chem. Commun. 1999, 1795; Bobbitt et al., Chem.Commun. 1996, 2745, Miyazawa und Endo, J. Molec. Catal. 49, 1988, L31; M. J. Verhoef et al. in "Studies in Surface Science and Catalysis", Vol. 125, S. 465 ff; D. Brunel et al. in "Studies in Surface Science and Catalysis", Vol. 125, S. 237 ff; Miyazawa und Endo, J. Polymer Sci., Polym. Chem. Ed. 23, 1985, 1527 und 2487; T. Osa, Chem. Lett. 1988, 1423). Beispiele sind PIPO, SiO₂-geträgertes TEMPO, Polystyrol- und Polyacrylsäure-geträgertes TEMPO.

Als Nitroxylverbindung besonders bevorzugt ist eine Verbindung der allgemeinen Formel I mit R¹, R², R³ und R⁴ in der Bedeutung CH₃
und R⁵ und R⁶ unabhängig voneinander in der Bedeutung Wasserstoff, OH, OR⁷, O-COR⁷, O-COOR⁷, OCONHR⁷, wobei R⁷ die Bedeutung eines linearen oder verzweigten, gesättigten oder ungesättigten C₁-C₂₀-Alkylrestes, oder die Bedeutung eines C₆-C₂₀-Aryl oder C₆-C₂₀-Aralkylrests hat,
-(O-CH₂-CH₂)ₙ-OR⁸, -(O-C₃H₆)ₙ-OR⁸, -(O-(CH₂)₄)ₙ-OR⁸, -O-CH₂-CHOH-CH₂-(O-CH₂-CH₂-)ₙ-OR⁸ mit R⁸ in der Bedeutung Wasserstoff,
C₁-C₁₀-Alkyl oder C₆-C₁₀-Aralkyl, mit n = 1 bis 100, oder CH₂-CHOH-CH₃ oder CH₂-CHOH-CH₂-CH₃,
NR⁹R¹⁰, NHCOR¹⁰, NHCOOR¹⁰, NHCONHR¹⁰, mit R⁹ und R¹⁰ unabhängig voneinander in der Bedeutung Wasserstoff, eines linearen oder verzweigten, gesättigten oder ungesättigten C₁-C₂₀-Alkylrestes, eines C₆-C₁₂-Cycloalkylrests oder eines C₆-C₂₀-Aryl oder C₆-C₂₀-Aralkylrests:

Als Nitroxylverbindung besonders bevorzugt ist eine Verbindung der allgemeinen Formel I mit R¹, R², R³ und R⁴ in der Bedeutung CH₃
worin R⁵ und R⁶ gemeinsam Ketal-Gruppierungen der Formeln O-CHR¹³CHR¹⁴-O oder O-CH₂CR¹⁵R¹⁶-CH₂-O mit R¹³, R¹⁴, R¹⁵ und R¹⁶ unabhängig voneinander in der Bedeutung Wasserstoff oder C₁-C₃-Alkyl bilden,
oder worin die Reste R⁵ und R⁶ zusammen die Bedeutung =O haben.

Als Nitroxylverbindung insbesondere bevorzugt ist eine Verbindung der allgemeinen Formel I mit R¹, R², R³ und R⁴ in der Bedeutung CH₃
worin R⁵ die Bedeutung Wasserstoff und R⁶ die Bedeutung Wasserstoff, OH, OR⁷, O-COR⁷, wobei R⁷ die Bedeutung eines linearen oder verzweigten gesättigten C₁-C₁₂-Alkylrestes, oder die Bedeutung eines Aryl- oder Benzylrests hat,
-(O-CH₂-CH₂)ₙ-OR⁸, -(O-C₃H₆)ₙ-OR⁸, -(O-(CH₂)₄)ₙ-OR⁸, -O-CH₂-CHOH-CH₂-(O-CH₂-CH₂-)ₙ-OR⁸ mit n = 1 bis 50 und R⁸ in der Bedeutung Wasserstoff oder CH₂-CHOH-CH₃ oder CH₂-CHOH-CH₂-CH₃
NR⁹R¹⁰, NHCOR¹⁰, mit R⁹ und R¹⁰ unabhängig voneinander in der Bedeutung eines linearen oder verzweigten gesättigten C₁-C₁₂-Alkylrestes, oder eines Aryl- oder Benzylrests.

Beispiele für einsetzbare Nitroxylverbindungen sind TEMPO, 4-Hydroxy-TEMPO, 4-Oxo-TEMPO, 4-Amino-TEMPO, 4-Acetamido-TEMPO, 4-Benzoyloxy-TEMPO, 4-Acetoxy-TEMPO und PIPO.

Die Nitroxylverbindung wird vorzugsweise in Mengen von 0,01 bis 50 Mol %, besonders bevorzugt in Mengen von 0,1 bis 20 Mol % bezogen auf die Menge an zu oxidierendem Alkohol eingesetzt. Sie kann in der organischen oder in der wässrigen Phase gelöst oder in geträgerter Form als eigenständige Phase eingesetzt werden.

Als Oxidationsmittel wird eine Verbindung ausgewählt aus der Gruppe Chlor, Brom, Iod, Hypochlorit, Chlorit, Chlorat, Hypobromid, Bromit, Bromat, Hypoiodid, Iodit, Iodat, Periodat, Fe(CN)₆³⁻ , N-Chlorverbindungen und deren Gemische eingesetzt. Bevorzugte Oxidationsmittel sind Hypochlorit und Hypobromit.

Bei salzartigen Oxidationsmitteln sind als Gegenionen Natrium, Kalium, Kalzium, Ammonium oder Tetraalkylammonium bevorzugt.

Das verwendete Oxidationsmittel kann auch in situ, z.B. elektrochemisch, durch Hydrolyse, z.B. durch Hydrolyse von N-Chlorverbindungen, oder durch Redoxreaktionen, wie bei Hypochlorit- oder Hypobromitlösungen durch Disproportionierung von Chlor bzw. Brom in alkalischer Lösung, oder durch die Redoxreaktion zwischen Hypochlorit und Bromid, die zur Bildung von Hypobromit führt, erzeugt werden.

Die verwendeten Oxidationsmittel werden vorzugsweise in Konzentrationen von 0,1 M bis zu ihrer jeweiligen Sättigungskonzentration eingesetzt.

Weitere mögliche Zusätze sind Halogen, z.B. Brom, und Salze, z.B. Alkali-, Erdalkali- oder Ammoniumhalogenide oder -sulfate, -carbonate, -hydrogencarbonate, Phosphorsäure und dessen Alkalimetall-, Erdalkalimetall- oder Ammoniumsalze oder Kohlendioxid.

Diese Zusätze können jeweils einer der beiden Phasen, ggf in Lösung, zugesetzt werden oder ggf. in gelöster Form als dritte Komponente dem kontinuierlichen Verfahren zugeführt werden.

Bei der Oxidation mit Hypochlorit ist der Zusatz von Brom oder Bromid in Mengen von 0,01 bis 100 Mol % bezogen auf die eingesetzte Menge Hypochlorit bevorzugt. Besonders bevorzugt ist der Zusatz von Brom oder Bromid in Mengen zwischen 1 und 50 Mol %.

Der pH-Wert der wässrigen Phase liegt vorzugsweise zwischen 6 und 14, besonders bevorzugt zwischen 7 und 12. Die Einstellung des gewünschten pH-Werts erfolgt vorzugsweise durch Zugabe eines Puffers, z.B. Natriumhydrogencarbonat, Dinatriumhydrogenphosphat oder Natriumdihydrogenphosphat oder einer Säure, z.B. Kohlendioxid, Phosphorsäure, Salzsäure oder Schwefelsäure, oder Base, z.B. NaOH.

Die Reaktionstemperatur beträgt vorzugsweise -10 bis +80°C, besonders bevorzugt -5°C bis +30°C.

Für die Oxidation sind vorzugsweise alle primären und sekundären Alkohole geeignet, die mit der wässrigen Phase gegebenenfalls unter Zusatz eines organischen Lösungsmittels eine Mischungslücke haben.

Primäre Alkohole werden zu Aldehyden oxidiert. Sekundäre Alkohole ergeben Ketone. Bei Verbindungen, die primäre und sekundäre Hydroxygruppen enthalten, werden bevorzugt die primären Hydroxygruppen oxidiert. Eine Übersicht über die Reaktivitätsfolge bei Anwesenheit mehrerer Hydroxygruppen gibt A.E.J. de Nooy, A.C. Besemer und H.V. Bekkum, Synthesis,1996, 1153.

Die zu oxidierenden Alkohole sind vorzugsweise lineare oder verzweigte gesättigte oder ungesättigte aliphatische Alkohole mit 1-60 C-Atomen, zyklische aliphatische gesättigte oder ungesättigte Alkohole mit 5-60 C-Atomen oder durch einen aromatischen Rest substituierte Alkohole mit 6-60 C-Atomen, wobei ein Wasserstoff oder mehrere Wasserstoffe oder ein Kohlenstoff oder mehrere Kohlenstoffe durch Heteroatome ersetzt sein können.

Besonders bevorzugt handelt es sich um lineare oder verzweigte gesättigte oder ungesättigte aliphatische Alkohole mit 1-30 C-Atomen, zyklische aliphatische gesättigte oder ungesättigte Alkohole mit 5-30 C-Atomen oder durch einen aromatischen Rest substituierte Alkohole mit 6-30 C-Atomen,
wobei ein Wasserstoff oder mehrere Wasserstoffe unabhängig voneinander durch F, Cl, Br, I, NO₂ ,ONO, CN, NC oder SCN ersetzt sein können,
und wobei eine CH₂-Gruppe oder mehrere CH₂-Gruppen unabhängig voneinander durch O, NH, C=O, CO₂, S, S=O, SO₂, P=O, PO₂ oder eine acetylenische C₂-Einheit ersetzt sein können,
und eine CH-Gruppe oder mehrere CH-Gruppen unabhängig voneinander durch N, B oder P ersetzt sein können,
und quartäre Kohlenstoffe durch Si, Sn oder Pb ersetzt sein können.

Insbesondere bevorzugt handelt es sich um lineare oder verzweigte gesättigte oder ungesättigte aliphatische Alkohole mit 1-30 C-Atomen, zyklische aliphatische gesättigte oder ungesättigte Alkohole mit 5-30 C-Atomen oder durch einen aromatischen Rest substituierte Alkohole mit 6-30 C-Atomen,
wobei ein Wasserstoff oder mehrere Wasserstoffe unabhängig voneinander durch F, Cl, Br, I, NO₂ ,ONO, CN, NC oder SCN ersetzt sein können,
und wobei eine CH₂-Gruppe oder mehrere CH₂-Gruppen unabhängig voneinander durch O, NH, C=O, CO₂, S , S=O, SO₂, P=O, PO₂ oder eine acetylenische C₂-Einheit ersetzt sein können,
und eine CH-Gruppe oder mehrere CH-Gruppen unabhängig voneinander durch N, B oder P ersetzt sein können,
und quartäre Kohlenstoffe durch Si, Sn oder Pb ersetzt sein können,
wobei mindestens eine dieser genannten Substitutionen mindestens einmal in 2 oder in 3-Position in bezug auf das die Hydroxygruppe-tragende C-Atom erfolgt sein müssen

Insbesondere bevorzugt sind ebenfalls Alkohole, deren Verteilungsverhältnis zwischen der organischen Phase und der wässrigen Phase mindestens 80 : 20 beträgt.

Insbesondere bevorzugt können die folgenden Hydroxyverbindungen oxidiert werden:
2-und 3-Hydroxycarbonsäureester, 2-und 3-Hydroxycarbonsäureamide, Carbonsäure-2-hydroxyalkylester, Carbonsäure-2-hydroxy-N-alkylamide, 1- oder 2-Hydroxyketone oder Dihydroxyketone, Hydroxymethyl-oder Hydroxyethylepoxide, Kohlensäure-O-alkyl-O'-2-hydroxyalkylester, Kohlensäure-2,2'-dihydroxyalkylester, 2- und 3-Hydroxylactone, Hydroxy-1,3-dioxolane, Hydroxy-1,3-dioxane, Hydroxy-1,4-dioxane, 2- und 3-Hydroxyimide, 2- und 3-Hydroxyalkylimide, 2- und 3-Hydroxylactame, 2- und 3-Hydroxyalkylurethane, 2-Hydroxyether, 2,2'Dihydroxyether Oligoethylenglycole, Oligopropylenglycole, Alkyl- oder Arylsulfonsäure-2-hydroxyalkylester, 2-Hydroxyalkylsulfonsäurealkylester, 2-Aminoalkohole, 2-Trialkylsilylalkohole und 2-Trialkylsilyloxyalkohole.
Beispiele für die genannten Verbindungsklassen sind: Isobuttersäure-2-hydroxyethylester, Isobuttersäure-3-hydroxypropylester, 2,2-Dimethyl-4-hydroxymethyl-1,3-dioxolan, Cyclohexylidenglycerin, Pantolacton, Dihydroxyaceton, Glycerin, 1,4-Butandiol, Trimethylsilylethanol, Tert.butyl-dimethylsilyloxyethanol, Phthalimidoethanol.

Mit dem erfindungsgemäßen Verfahren wurde 2-n-Butyryloxyacetaldehyd aus Buttersäure-2-hydroxyethylester in 80 proz. Ausbeute bei vollständigem Umsatz erhalten.
Mit dem erfindungsgemäßen Verfahren ist es ebenso möglich, instabile Substanzen wie den bislang nicht präparativ darstellbaren Trimethylsilylacetaldehyd zu synthetisieren.

Die folgenden Beispiele dienen der weiteren Erläuterung der Erfindung.

### Beispiel 1: 2-n-Butyryloxyacetaldehyd

Es wurden folgende Lösungen hergestellt:
Lösung 1: 700 g (5,30 mol) 2-n-Butyryloxyethanol, 43 g (0,28 mol) TEMPO und 47.0 g (0,294 mol) Brom in 10 l Methylenchlorid.
Lösung 2: 4,20 l Natriumhypochloritlösung (technische Bleichlauge) wurde mit gasf. CO₂ auf einen pH-Wert von 9,5 eingestellt. Gehalt ca. 1,7 M.
Mittels Dosierpumpen wurden die Lösungen 1 und 2 aus zwei Reservoirs (Volumen jeweils 10 l) über ein statisches Mischelement in ein als Spirale gewickeltes 50 m langes in einem Eisbad gekühltes V4A-Rohr (Innendurchmesser 2 mm, Außendurchmesser 3,2 mm) gepumpt. Die Pumprate betrug für die Lösung 1 133 ml/min und für die Lösung 2 55 ml/min. Probennahme nach 25 min ergab vollständigen Umsatz. Die Reaktionsmischung wurde in einem 20 l-Behälter aufgefangen, die Methylenchloridlösung abgelassen, mit 0,5 l 10 proz. HCl, 0,5 l 10 proz. Natriumthiosulfatlösung und mit 0,5 l Wasser gewaschen und fraktioniert destilliert. Ausbeute 70 % 2-n-Butyryloxyacetaldehyd.

### Beispiel 2: 2-n-Butyryloxyacetaldehyd

Es wurden folgende Lösungen hergestellt:
Lösung 1: 494 g (3,74 mol) 2-n-Butyryloxyethanol und 35,4 g (0,205 mol) 4-Hydroxy-TEMPO in 7,27 1 Methylenchlorid.
Lösung 2: 4,20 l Natriumhypochloritlösung (technische Bleichlauge) wurde mit gasf. CO₂ auf einen pH-Wert von 9,5 eingestellt. Gehalt ca. 2,1 M.
Lösung 3: 84,9 g NaBr in 313 ml Wasser.
Mittels Dosierpumpen wurden die Lösungen 1, 2 und 3 aus Reservoirs über ein statisches Mischelement in ein als Spirale gewickeltes 20 m langes in einem Eisbad gekühltes Titan-Rohr (Innendurchmesser 3 mm, Außendurchmesser 4,1 mm) gepumpt. Die Pumprate betrug für die Lösung 1 48 l/h, für die Lösung 2 13 l/h und die Lösung 3 0,5 l/h. Die Reaktionsmischung wurde in einem 20 l-Behälter aufgefangen, die Methylenchloridlösung abgelassen, mit 0,5 l 10 proz. HCl, 0,5 l 10 proz. Natriumthiosulfatlösung und mit 0,5 l Wasser gewaschen und fraktioniert destilliert. Ausbeute 80 % 2-n-Butyryloxyacetaldehyd.

### Beispiel 3: 2-n-Butyryloxyacetaldehyd

Der Versuch wurde wie in Beispiel 2 ausgeführt. Der pH-Wert der Bleichlauge wurde auf 11 eingestellt. Ausbeute 81 % 2-n-Butyryloxyacetaldehyd.

### Beispiel 4: 2-n-Butoxyacetaldehyd

2-n-Butoxyethanol wurde wie in Beispiel 2 umgesetzt. Ausbeute: 80 %, Sdp.: 40°C (12 mb).

### Beispiel 5: Pivalinaldehyd

Neopentylalkohol wurde wie in Beispiel 2 umgesetzt. Lösung 1 bestand aus 363 g Neopentylalkohol und 48 g 4-Acetamido-TEMPO in 1 l Methylenchlorid, Die Pumprate betrug für die Lösung 1 30 l/h, für die Lösung 2 22 l/h und die Lösung 3 0,5 l/h. Ausbeute: 93 % (¹H-NMR-Standardanalyse).

### Beispiel 6: Trimethylsilylacetaldehyd

2-(Trimethylsilyl)ethanol wurde wie in Beispiel 2 umgesetzt. Ausbeute in Lösung 71 % (¹H-NMR-Standardanalyse). ¹H-NMR (CDCl₃/CH₂Cl₂): δ=0,48 (s, 3 CH₃), 2, 30 (s, CH₂), 9,67 (s, CHO); Lagerung bei Raumtemp. führt zur langsamen Zersetzung der Substanz.

### Beispiel 7: Tert.-butyl-dimethylsilyloxyacetaldehyd

2-(Tert.-butyldimethylsilyloxy)ethanol wurde wie in Beispiel 2 umgesetzt. Lösung 1 bestand aus 108 g (0,610 mol) 2-(Tert.butyl-dimethylsilyloxy)ethanol und 5,8 g 4-Hydroxy-TEMPO in 400 ml Ethylacetat. Durchflussraten: 48 l/h für Lösung 1, 30 l/h für Lösung 2. Tert.-butyl-dimethylsilyloxyacetaldehyd wurde durch fraktionierte Destillation der Ethylacetat-Phase gewonnen. Sdp.: 74-80°C/22 mbar

### Beispiel 8: N-(2-Oxoethyl)phthalimid

Die Darstellung aus N-(2-Hydroxyethyl)phthalimid erfolgte wie in Beispiel 2, die Kühlung erfolgte mit Leitungswasser. Ausbeute: 74 %, Schmp. 114°C (aus Methylenchlorid).

### Beispiel 9: 2-n-Butyryloxyacetaldehyd (Vergleichsbeispiel)

20,0 g (151 mmol) 2-n-Butyryloxyethanol wurden in 300 ml Methylenchlorid gelöst und mit 1,30 g (8.32 mmol) TEMPO versetzt. Die Lösung wurde auf -10°C gekühlt und nach Zugabe einer Lösung von 2,0 g (16,8 mmol) KBr in 9 ml Wasser unter starkem Rühren und Kühlung innerhalb einer Stunde mit 150 g (278 mmol) technischer Bleichlauge, deren pH-Wert mit festem Natriumhydrogencarbonat auf ca. 9,5 eingestellt wurde, versetzt. Die Temperatur wurde bei der Zugabe auf ca. 0°C gehalten. Nach der Zugabe wurden die Phasen getrennt, die organische Phase nacheinander mit 15 ml 10 proz. HCl, 15 ml 10 proz. Natriumthiosulfatlösung und mit 15 ml Wasser gewaschen. ¹H-NMR-Standardanalyse ergab eine Ausbeute von 37 % 2-Isobutyroxyacetaldehyd in der Dichlormethanphase. Die folgenden weiteren Produkte wurden ebenso bestimmt: 12 % 2-n-Butyryloxyessigsäure-2-n-butyryloxyethylester (Methylenchloridphase), 8 % 2-n-Butyryloxyessigsäure (Methylenchloridphase), 33 % 2-n-Butyryloxyessigsäure (Wasserphase), 4 % Buttersäure (Wasserphase).

### Beispiel 10: 2-n-Butyryloxyacetaldehyd (Vergleichsbeispiel)

40,0 g (303 mmol) 2-n-Butyryloxyethanol wurden analog zu Beispiel 9 umgesetzt und aufgearbeitet. Die Reaktionszeit betrug 100 min.
¹H-NMR-Standardanalyse ergab eine Ausbeute von 20 % 2-n-Butyryloxyacetaldehyd, 45 % 2-n-Butyryloxyessigsäure-2-nbutyryloxyethylester, 22 % 2-n-Butyryloxyessigsäure und 8 % Buttersäure in der Methylenchloridphase.

### Beispiel 11: 2-n-Butyryloxyacetaldehyd (Vergleichsbeispiel)

Es wurden die Lösungen 1 und 2 aus Beispiel 1 verwendet.
Die beiden Lösungen wurden synchron im Volumenverhältnis 2,4 : 1 in einem hochtourig gerührten Verweilzeitkessel (V = 250 ml) gemischt. Durch gleichzeitiges Ablassen der Lösung wurde die mittlere Verweildauer auf 20 min. eingestellt. Ausbeute an n-Butyryloxyacetaldehyd in Methylenchlorid: 19 %, nicht-umgesetzter Alkohol 15 %, n-Butyryloxyessigsäure 32 % (Methode: GC- und ¹H-NMR-Standardanalyse).

### Beispiel 12: Pivalinaldehyd (Vergleichsbeispiel)

Es wurden die Lösungen 1 und 2 aus Beispiel 5 verwendet. Analog zu Beispiel 11 wurde Neopentylalkohol umgesetzt. Ausbeute an Pivalinaldehyd 47 %, nicht-umgesetzter Alkohol 25 % (Methode: GC- und ¹H-NMR-Standardanalyse).

## Patentansprüche

1. Verfahren zur Oxidation eines Alkohols zu einem Aldehyd oder einem Keton unter Katalyse einer Nitroxylverbindung wobei der zu oxidierende Alkohol in einer organischen flüssigen Phase in Gegenwart einer Nitroxylverbindung mit einer das Oxidationsmittel enthaltenden wässrigen Phase umgesetzt wird, **dadurch gekennzeichnet, dass** die Umsetzung mit einer Kontaktzeit der Phasen zwischen 0,1 s und maximal 15 min. und bei einer intensiven Durchmischung der Phasen kontinuierlich erfolgt.

2. Verfahren nach Anspruch 1, **dadurch gekennzeichnet, dass** die Kontaktzeit der Phasen 1 s bis 5 min, bevorzugt 1s bis 2 min beträgt.

3. Verfahren nach Anspruch 1 oder 2, **dadurch gekennzeichnet, daß** die intensive Durchmischung durch die Ausbildung einer turbulenten Strömung erreicht wird.

4. Verfahren nach Anspruch 3, **dadurch gekennzeichnet, daß** die turbulente Strömung eine Reynolds`sche Zahl Re zwischen 800 und 20000 besitzt.

5. Verfahren nach einem der Ansprüche 1 bis 4, **dadurch gekennzeichnet, daß** die organische flüssige Phase aus dem zu oxidierenden Alkohol und gegebenenfalls einem oder mehreren organischen Lösungsmitteln besteht.

6. Verfahren nach einem der Ansprüche 1 bis 5, **dadurch gekennzeichnet, daß** der zu oxidierende Alkohol in Konzentrationen zwischen 0,1 und 100 Gew.% bezogen auf die organische Lösung eingesetzt wird.

7. Verfahren nach einem der Ansprüche 1 bis 6, **dadurch gekennzeichnet, daß** die als Oxidationskatalysator eingesetzte Nitroxylverbindung eine Di-tert.-alkylnitroxylverbindung ist.

8. Verfahren nach Anspruch 7, **dadurch gekennzeichnet, daß** die Nitroxylverbindung ein Verbindung der allgemeinen Formel I ist, 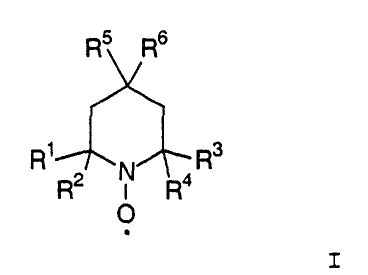 worin die Reste R¹, R², R³ und R⁴ unabhängig voneinander C₁-C₁₂-Alkyl oder C₂-C₁₂-Alkenyl oder C₆-C₁₂-Aryl oder Aralkyl bedeuten, und die Reste R⁵ und R⁶ unabhängig voneinander folgende Bedeutung haben: Wasserstoff, OH, CN, Halogen,
linear oder verzweigt, gesättigt oder ungesättigt C₁-C₂₀-Alkyl-, C₆-C₂₀-Aryl, C₆-C₂₀-Hetaryl oder C₆-C₂₀-Aralkyl,
OR⁷, O-COR⁷, O-COOR⁷, OCONHR⁷ ,
COOH, COR⁷, COOR⁷, CONHR⁷ wobei R⁷ die Bedeutung eines linearen oder verzweigten, gesättigten oder ungesättigten C₁-C₂₀-Alkylrestes, oder die Bedeutung eines C₆-C₂₀-Aryl, C₃-C₂₀-Hetaryl oder C₆-C₂₀-Aralkylrests hat,
-(C-CH₂-CH₂)ₙ-OR⁸ , -(O-C₃H₆)ₙ-OR⁸, -(O-(CH₂)₄)ₙ-OR⁸, -O-CH₂-CHOH-CH₂-(O-CH₂-CH₂)ₙ-OR⁸, mit R⁸ in der Bedeutung Wasserstoff, C₁-C₂₀-Alkyl, C₆-C₂₀-Aralkyl, mit n = 1 bis 100 , oder CH₂-CHOH-CH₃ oder CH₂-CHOH-CH₂-CH₃ ,
NR⁹R¹⁰, NHCOR⁹, NHCOOR⁹, NHCONHR⁹, mit R⁹ und R¹⁰ unabhängig voneinander in der Bedeutung eines linearen oder verzweigten, gesättigten oder ungesättigten C₁-C₂₀-Alkylrestes, eines C₆-C₁₂-Cycloalkylrests, oder eines C₆-C₂₀-Aryl , C₃-C₂₀ Hetaryl oder C₆-C₂₀-Aralkylrests,
wobei die Reste R⁵ und R⁶ auch zu einem Ring verknüpft sein können,
und wobei die Reste R⁵ und R⁶ ihrerseits auch mit COOH, OH, SO₃H, CN, Halogen, prim., sek., tert. Amino oder quart. Ammonium substituiert sein können, oder
die Reste R⁵ und R⁶ zusammen auch die Bedeutung =O, =NR¹¹, =N-OR¹¹, =N-N=CR¹¹R¹² mit R¹¹ und R¹² unabhängig in der Bedeutung Wasserstoff, C₁-C₂₀-Alkyl oder C₆-C₂₀-Aralkyl haben können.
Weiterhin bevorzugt handelt es sich bei der Nitroxylverbindung um zwei Moleküle der Formel I, die über eine Brücke =N-N= in 4-Position verknüpft sind.

9. Verfahren nach einem der Ansprüche 1 bis 8, **dadurch gekennzeichnet, daß** die Nitroxylverbindung in Mengen von 0,01 bis 50 Mol %, besonders bevorzugt in einer Menge von 0,1 bis 20 Mol % bezogen auf die Menge an zu oxidierendem Alkohol eingesetzt wird.

10. Verfahren nach einem der Ansprüche 1 bis 9, **dadurch gekennzeichnet, daß** als Oxidationsmittel eine Verbindung, ausgewählt aus der Gruppe Chlor, Brom, Jod, Hypochlorit, Chlorit, Chlorat, Hypobromid, Bromit, Bromat, Hypoiodid, Iodit, Iodat, Periodat, Fe(CN)₆³⁻, N-Chlorverbindungen und deren Gemische eingesetzt wird.

11. Verfahren nach einem der Ansprüche 1 bis 10, **dadurch gekennzeichnet, daß** es bei einer Reaktionstemperatur von -10 bis +80°C, bevorzugt von -5°C bis +30°C verläuft.

12. Verfahren nach einem der Ansprüche 1 bis 11, **dadurch gekennzeichnet, dass** das Verfahren in einem Rohrreaktor ausgeführt wird.

## Claims

1. Process for the oxidation of an alcohol to an aldehyde or a ketone in the presence of a nitroxyl compound as catalyst, in which the alcohol to be oxidized in an organic liquid phase is reacted in the presence of a nitroxyl compound with an aqueous phase comprising the oxidant, **characterized in that** the reaction is carried out continuously at a contact time of the phases of from 0.1 s to a maximum of 15 minutes and with intensive mixing of the phases.

2. Process according to Claim 1, **characterized in that** the contact time of the phases is from 1 s to 5 minutes, preferably from 1 s to 2 minutes.

3. Process according to Claim 1 or 2, **characterized in that** the intensive mixing is achieved by establishment of turbulent flow.

4. Process according to Claim 3, **characterized in that** the turbulent flow has a Reynolds number Re of from 800 to 20 000.

5. Process according to any of Claims 1 to 4, **characterized in that** the organic liquid phase comprises the alcohol to be oxidized and, if desired, one or more organic solvents.

6. Process according to any of Claims 1 to 5, **characterized in that** the alcohol to be oxidized is used in concentrations of from 0.1 to 100% by weight based on the organic solution.

7. Process according to any of Claims 1 to 6, **characterized in that** the nitroxyl compound used as oxidation catalyst is a di-tert-alkylnitroxyl compound.

8. Process according to Claim 7, **characterized in that** the nitroxyl compound is a compound of the formula I, 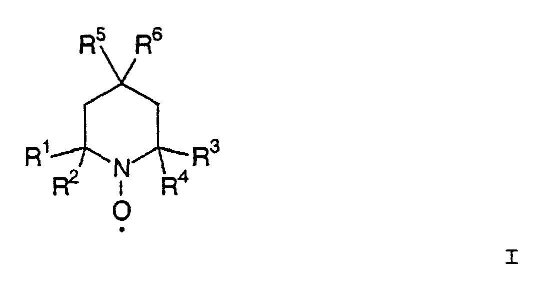 where the radicals R¹, R², R³ and R⁴ are each, independently of one another, C₁-C₁₂-alkyl or C₂-C₁₂-alkenyl or C₆-C₁₂-aryl or aralkyl and the radicals R⁵ and R⁶ each have, independently of one of another, one of the following meanings: hydrogen, OH, CN, halogen,
linear or branched, saturated or unsaturated C₁-C₂₀-alkyl, C₆-C₂₀-aryl, C₆-C₂₀-hetaryl or C₆-C₂₀-aralkyl,
OR⁷, O-COR⁷, O-COOR⁷, OCONHR⁷,
COOH, COR⁷, COOR⁷, CONHR⁷ where R⁷ is a linear or branched, saturated or unsaturated C₁-C₂₀-alkyl radical or a C₆-C₂₀-aryl, C₃-C₂₀-hetaryl or C₆-C₂₀-aralkyl radical,
-(O-CH₂-CH₂)ₙ-OR⁸, -(O-C₃H₆)ₙ-OR⁸, -(O-(CH₂)₄)ₙ-OR⁸, -O-CH₂-CHOH-CH₂-(O-CH₂-CH₂-)ₙ-OR⁸, where R⁸ is hydrogen, C₁-C₂₀-alkyl, C₆-C₂₀-aralkyl, and n = 1 to 100, or CH₂-CHOH-CH₃ or CH₂-CHOH-CH₂-CH₃,
NR⁹R¹⁰, NHCOR⁹, NHCOOR⁹, NHCONHR⁹ where R⁹ and R¹⁰ are each, independently of one another, a linear branched, saturated or unsaturated C₁-C₂₀-alkyl radical, a C₆-C₁₂-cycloalkyl radical or a C₆-C₂₀-aryl, C₃-C₂₀-hetaryl or C₆-C₂₀-aralkyl radical, where the radicals R⁵ and R⁶ may also be joined to form a ring and,
the radicals R⁵ and R⁶ may in turn be substituted by COOH, OH, SO₃H, CN, halogen, primary, secondary or tertiary amino or quaternary ammonium or
the radicals R⁵ and R⁶ may together also represent =O, =NR¹¹, =N-OR¹¹, =N-N=CR¹¹R¹², where R¹¹ and R¹² are each, independently of one another, hydrogen, C₁-C₂₀-alkyl or C₆-C₂₀-aralkyl,
with preference also being given to the nitroxyl compound being formed by two molecules of the formula I which are linked in the 4 position by a bridge =N-N=.

9. Process according to any of Claims 1 to 8, **characterized in that** the nitroxyl compound is used in an amount of from 0.01 to 50 mol%, particularly preferably in an amount of from 0.1 to 20 mol%, based on the amount of alcohol to be oxidized.

10. Process according to any of Claims 1 to 9, **characterized in that** the oxidant used is a compound selected from the group consisting of chlorine, bromine, iodine, hypochlorite, chlorite, chlorate, hypobromite, bromite, bromate, hypoiodite, iodite, iodate, periodate, Fe(CN)₆³-, N-chloro compounds and mixtures thereof.

11. Process according to any of Claims 1 to 10 carried out at a reaction temperature of from -10 to +80°C, preferably from -5°C to +30°C.

12. Process according to any of Claims 1 to 11 carried out in a tube reactor.

## Revendications

1. Procédé pour l'oxydation d'un alcool en un aldéhyde ou une cétone avec catalyse d'un composé nitroxyle, l'alcool à oxyder étant transformé dans une phase liquide organique en présence d'un composé nitroxyle avec une phase aqueuse contenant l'oxydant, **caractérisé en ce que** la transformation est réalisée en continu avec un temps de contact des phases entre 0,1 s et au maximum 15 minutes et en mélangeant les phases intensivement.

2. Procédé selon la revendication 1, **caractérisé en ce que** le temps de contact des phases est de 1 seconde à 5 minutes, de préférence de 1 seconde à 2 minutes.

3. Procédé selon la revendication 1 ou 2, **caractérisé en ce que** le mélange intensif est réalisé par la réalisation d'un écoulement turbulent.

4. Procédé selon la revendication 3, **caractérisé en ce que** l'écoulement turbulent présente un indice de Reynolds Re entre 800 et 20000.

5. Procédé selon l'une quelconque des revendications 1 à 4, **caractérisé en ce que** la phase liquide organique est constituée de l'alcool à oxyder et le cas échéant d'un ou de plusieurs solvants organiques.

6. Procédé selon l'une quelconque des revendications 1 à 5, **caractérisé en ce que** l'alcool à oxyder est utilisé en des concentrations entre 0,1 et 100% en poids par rapport à la solution organique.

7. Procédé selon l'une quelconque des revendications 1 à 6, **caractérisé en ce que** le composé nitroxyle utilisé comme catalyseur d'oxydation est un composé di-tert-alkylnitroxyle.

8. Procédé selon la revendication 7, **caractérisé en ce que** le composé nitroxyle est un composé de formule générale I 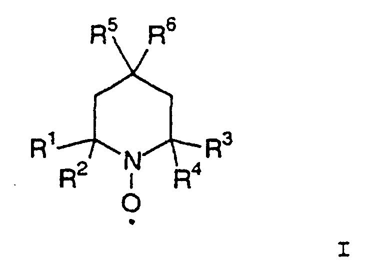 dans laquelle les radicaux R¹, R², R³ et R⁴ signifient indépendamment l'un de l'autre alkyle en C₁ à C₁₂ ou alcényle en C₂ à C₁₂ ou aryle en C₆ à C₁₂ ou aralkyle et les radicaux R⁵ et R⁶ présentent, indépendamment l'un de l'autre, la signification suivante : hydrogène, OH, CN, halogène,
alkyle en C₁ à C₂₀ linéaire ou ramifié, saturé ou insaturé, aryle en C₆ à C₂₀, hétéroaryle en C₆ à C₂₀ ou aralkyle en C₆ à C₂₀,
OR⁷, O-COR⁷, O-COOR⁷, OCONHR⁷,
COOH, COR⁷, COOR⁷, CONHR⁷, R⁷ ayant la signification d'un radical alkyle en C₁ à C₂₀ linéaire ou ramifié, saturé ou insaturé ou la signification d'un radical aryle en C₆ à C₂₀, hétéroaryle en C₃ à C₂₀ ou aralkyle en C₆ à C₂₀, -(C-CH₂-CH₂)ₙ-OR⁸, -(O-C₃H₆)ₙ-OR⁸, -(O-(CH₂)₄)ₙ-OR⁸, -O-CH₂-CHOH-CH₂-(O-CH₂-CH₂-)ₙ-OR⁸, avec R⁸ ayant la signification d'un hydrogène, alkyle en C₁ à C₂₀, aralkyle en C₆ à C₂₀, avec n = 1 à 100, ou CH₂-CHOH-CH₃ ou CH₂-CHOH-CH₂-CH₃,
NR⁹R¹⁰, NHCOR⁹, NHCOOR⁹, NHCONHR⁹, avec R⁹ et R¹⁰ ayant indépendamment l'un de l'autre la signification d'un radical alkyle en C₁ à C₂₀ linéaire ou ramifié, saturé ou insaturé, d'un radical cycloalkyle en C₆ à C₁₂ ou aryle en C₆ à C₂₀, hétéroaryle en C₃ à C₂₀ ou aralkyle en C₆ à C₂₀,
les radicaux R⁵ et R⁶ pouvant également être liés dans un cycle
et les radicaux R⁵ et R⁶ pouvant à leur tour également être substitués par COOH, OH, SO₃H, CN, halogène, amino primaire, secondaire, tertiaire ou ammonium quaternaire ou
les radicaux R⁵ et R⁶ pouvant également avoir ensemble la signification =O, =NR¹¹, =N-OR¹¹, =N-N=CR¹¹R¹² avec R¹¹ et R¹² pouvant présenter indépendamment la signification hydrogène, alkyle en C₁ à C₂₀ ou aralkyle en C₆ à C₂₀.
Il s'agit également de préférence, dans le cas du composé nitroxyle, de deux molécules de formule I, qui sont liées via un pont =N-N= en 4^{ème} position.

9. Procédé selon l'une quelconque des revendications 1 à 8, **caractérisé en ce que** le composé nitroxyle est utilisé en des quantités de 0,01 à 50% en mole, de manière particulièrement préférée en une quantité de 0,1 à 20% en mole par rapport à la quantité d'alcool à oxyder.

10. Procédé selon l'une quelconque des revendications 1 à 9, **caractérisé en ce qu'**on utilise comme oxydant un composé choisi dans le groupe chlore, brome, iode, hypochlorite, chlorite, chlorate, hypobromure, bromite, bromate, hypoiodure, iodite, iodate, periodate, Fe(CN)₆³-, des composés N-chlorés et leurs mélanges.

11. Procédé selon l'une quelconque des revendications 1 à 10, **caractérisé en ce qu'**il se déroule à une température de réaction de -10 à +80°C, de préférence de -5°C à +30°C.

12. Procédé selon l'une quelconque des revendications 1 à 11, **caractérisé en ce que** le procédé est réalisé dans un réacteur tubulaire.
